# FASCICULE DE BREVET EUROPEEN

(11) **EP 4 409 261 B1**
(45) Date de publication et mention de la délivrance du brevet: **11.02.2026**
(21) Numéro de dépôt: 22777724.0
(22) Date de dépôt: 27.09.2022
(51) Int. Cl.: G01N 15/14, G01N 33/68

(54) **METHODE DE DETECTION, DE QUANTIFICATION ET DE DISCRIMINATION DES LEVURES BRETTANOMYCES SPP ET DES AUTRES LEVURES CONTENUES DANS UN SUBSTRAT LIQUIDE ORGANIQUE CONTENANT DES SUCRES FERMENTESCIBLES**
VERFAHREN ZUM NACHWEIS, ZUR QUANTIFIZIERUNG UND CHARAKTERISIERUNG VON BRETTANOMYCES SPP. IN EINEM ORGANISCHEN FLÜSSIGEN SUBSTRAT MIT FERMENTIERBAREN ZUCKERN ENTHALTENE UND ANDERE HEFEN
METHOD FOR DETECTING, QUANTIFYING AND CHARACTERISING BRETTANOMYCES SPP. AND OTHER YEASTS CONTAINED IN AN ORGANIC LIQUID SUBSTRATE CONTAINING FERMENTABLE SUGARS

(30) Priorité: 28.09.2021 FR 2110234
(43) Date de publication de la demande: 07.08.2024
(73) Titulaire: MD Invention Oeno, 11100 Montredon-des- Corbières (FR)
(72) Inventeur: DUBERNET, Matthieu, 11100 Narbonne (FR)
(74) Mandataire: Alatis
(86) Numéro de dépôt international: PCT/IB2022/059194
(87) Numéro de publication internationale: WO 2023/053002

(56) Documents cités:
- BRANCO PATRÍCIA ET AL: "A simple procedure for detecting Dekkera bruxellensis in wine environment by RNA-FISH using a novel probe", INTERNATIONAL JOURNAL OF FOOD MICROBIOLOGY, ELSEVIER BV, NL, vol. 314, 30 October 2019 (2019-10-30), XP086034169, ISSN: 0168-1605, [retrieved on 20191030], DOI: 10.1016/J.IJFOODMICRO.2019.108415
- ORO L. ET AL: "Evaluation of damage induced by Kwkt and Pikt zymocins against Brettanomyces/Dekkera spoilage yeast, as compared to sulphur dioxide", vol. 121, no. 1, 29 April 2016 (2016-04-29), GB, pages 207 - 214, XP055915796, ISSN: 1364-5072, Retrieved from the Internet <URL:https://api.wiley.com/onlinelibrary/tdm/v1/articles/10.1111%2Fjam.13121> DOI: 10.1111/jam.13121
- THORNTON ROY ET AL: "Evaluation of Yeast Viability and Concentration during Wine Fermentation Using Flow Cytometry", 1 April 2002 (2002-04-01), XP055915539, Retrieved from the Internet <URL:https://www.bdj.co.jp/flow-reagent/articles/hkdqj200000hsnkc-att/ApNote_23-6289-01.pdf> [retrieved on 20220426]

## Description

### Domaine technique

La présente invention concerne une méthode permettant avec un même échantillon et un seul passage dans un cytomère, la détection, la quantification et la discrimination des levures *Brettanomyces* et des autres levures, notamment *Saccharomyces spp* contenues dans un substrat liquide organique contenant des sucres fermentescibles.

### Art antérieur

Les levures du genre *Brettanomyces* sont capables de produire des composés produisant entre autres, des odeurs d'écurie. *Brettanomyces* est relativement résistante à l'alcool et à des pH bas. Dans la majorité des cas, elle est sensible au SO₂, certaines souches peuvent y être particulièrement résistantes. Les levures *Brettanomyces* sont de plus petite taille comparées à *Saccharomyces cerevisiae* mais elles restent difficiles à identifier du fait d'un important polymorphisme. De plus, dans un milieu complexe comme le vin, le vinaigre, ou un moût, par exemple, leur discrimination est encore plus compliquée du fait des interactions avec le milieu. La plus commune des levures *Brettanomyces* est *Brettanomyces bruxellensis* qui comprend un genre sporulant appelé *Dekkera bruxellensis.* Ces levures produisent notamment du 4-éthyl-phénol, lequel est stable dans le vin et lui donne une odeur d'écurie qu'il est souhaitable d'éviter. Le même phénomène peut apparaître dans d'autres jus fermentés et éventuellement alcoolisés comme le cidre, la bière, la téquila, le kombucha, le kéfir, par exemple. *Brettanomyces spp* étant présente dans le milieu environnant, elle est susceptible de contaminer n'importe quel substrat liquide contenant des sucres fermentescibles dans lequel elle peut se développer. Les levures *Brettanomyces* peuvent se présenter sous plusieurs états : vivante (donc active et produisant le composé précité), morte (non inactive et ne produisant pas le composé précité) ou latente. Les cellules latentes sont susceptibles de redevenir active et donc de produire entre autres, du 4-éthyl-phénol lors du vieillissement du vin, par exemple. Les cellules à l'état latent ne sont détectables par mise en culture du substrat ce qui signifie que la mise en culture d'un échantillon de substrat ne permet pas de garantir dans le futur l'absence de fragrances indésirables.

La publication intitulée « The application of flow cytometry in microbiological monitoring during winemaking: two case studies » de R. Guzzon & al et publiée dans la revue Ann Microbiol ((2015) 65/1865-1878) indique qu'il est possible de différentier par cytométrie en flux une cellule vivante d'une cellule morte dans une culture de *S*. *cerevisiae* pure ou dans un vin dans lequel on a ajouté un mélange de sucre et de *S*. *cerevisiae.* La méthode appliquée au le vin avant ajout du mélange précité pour une seconde fermentation ne donne aucun résultat. Un mélange de marqueurs est utilisé dans cette méthode : le mélange cFDA combiné à l'iodure de propidium.

La publication intitulée « Rapid detection of viable yeasts and bacteria in wine by flow cytometry » de Malacrino & al publiée dans la revue Journal of Microbiological Methods (45 (2001) 127-134) indique qu'il est possible de déterminer le nombre de levures et de bactéries malolactiques dans du jus de Pinot noir dans lequel on a jouté des levures pré cultivées dans un milieu particulier (1% d'extrait de levure +2% de peptone +2% de dextrose) par cytométrie en flux. Le meilleur marqueur est le mélange fluorescéine cFDA. La même méthode appliquée sur un échantillon de vin ne donne pas des résultats cohérents avec les résultats obtenus par comptage sur des cultures. Le vin doit donc être préalablement lavé avant la mesure. La publication suggère que cette méthode pourrait être utilisée pour la détection de levures non désirables telles que *Brettanomyces spp.*

La publication intitulée « Survival and metabolism of hydroxycinnamic acids by Dekkera bruxellensis in monovarietal wines » de Nine de Lima, publiée dans la revue Food microbiology en février 2021 (volume 93) indique qu'il est possible de mesurer la population de *Dekkera bruxellensis* dans du vin préalablement inoculé avec une souche de *Dekkera bruxellensis* par cytométrie en flux. Deux marqueurs ont été utilisés en combinaison : l'iodure de propidium et SYTO 9 ^{®}.

La publication intitulée « Specific Identification and Quantification of the Spoilage Microorganism Brettanomyces in Wine by Flow Cytometry: A Useful Tool for Winemakers », de H. Alexandre & al, publiée en ligne le 11 février 2010 dans la revue Wiley Interscience décrit une méthode d'identification et de quantification de Brettanomyces dans le vin par spectrométrie de flux par utilisation de la méthode d'hybridation de fluorescence in situ. Le marqueur fluorescents utilisé (Alexa Fluor^{®} 488) cible des séquences particulières d'acides aminés qui correspondent à l'ARN de Brettanomyces. La membrane de Brettaomyces contenue dans le vin est d'abord perméabilisée ce qui ne permet pas de différencier les cellules mortes des vivantes ou latentes. Le seuil de détection est de 102 cellules /mL.

La publication intitulée « A simple procedure for detecting Dekkera bruxellensis in wine environment by RNA-FISH using a novel probe » de Branco P & al publiée dans la revue international Journal of Food Microbiology Elsevier en 2019 décrit une méthode de détection de *D*. *bruxellensis (Brettanomyces dekkera bruxellensis)* dans du vin à l'aide d'une sonde fluorescente spécifique qui cible une séquence particulière de l'ARN ribosomique de *D*. *bruxellensis.* Cette sonde permet une bonne détection si la quantité d'ARN ribosomique est assez importante afin d'obtenir un signal fluorescent d'une intensité suffisante. Selon cette méthode, les échantillons sont incubés pendant 2 heures à 46°C. La diode rouge (680/30) est utilisée pour l'excitation de l'échantillon. Les membranes cellulaires sont perméabilisées (durée 1 heure) ce qui ne permet pas de distinguer ensuite les cellules mortes, des cellules vivantes.

La publication intitulée « Evaluation of damage induced by Kwt and Pikt zymocins against Brettanomyces/Dekkera spoilage yeast, as compared to sulphur dioxide » de Ora L. et al et publiée dans la revue Journal of applied microbiology en 2016 indique d'utiliser de l'iodure de propidium pour détecter *Dekkera bruxellensis.* Ce document traite de la toxicité de deux zymocines sur *Brettanomyces* mais ne décrit pas une méthode de détection de *Brettanomyces spp.*

Le document intitulé « evaluation of yeast viability and concentration during wine fermentation using flow cytometry » de Thornton, p 209 du livre « Cultivability, mortality and metabolic activity » indique qu'il est possible de détecter des levures dans le vin et de déterminer si ces levures sont vivantes. Par contre, ce document ne décrit pas la possibilité de discriminer *Brettanomyces spp* des autres levures.

### Problème Technique

Un but de la présente invention est de proposer une méthode par cytométrie en flux qui permet de déterminer, de quantifier et de discriminer les levures *Brettanomyces spp* des autres levures et notamment des levures *Saccharomyces spp* dans un substrat liquide contenant des sucres fermentescibles et éventuellement des particules fines de la taille des bactéries ou levures.

Un autre but de la présente invention est de proposer une méthode par cytométrie en flux qui permet de discriminer et de quantifier les cellules vivantes des levures *Brettanomyces spp,* les cellules mortes des levures *Brettanomyces* spp et les cellules latentes de levures *Brettanomyces spp.*

Un autre but de la présente invention est de proposer une méthode qui soit rapide à mettre en œuvre et nécessite en particulier un temps d'incubation réduit.

Un autre but de la présente invention est de proposer une méthode qui soit applicable à un produit fini, comme du vin, du cidre, de la bière, du vinaigre ou du jus de fruit, éventuellement mis en cuve ou en bouteille et apte à la vente.

Un autre but de l'invention est de proposer une méthode qui permet avec une analyse d'un même échantillon de dénombrer également les bactéries et éventuellement leur état (morte, latente ou vivante)

### Brève description de l'invention

La présente invention concerne une méthode de détection, de quantification et de discrimination par cytométrie en flux des cellules des levures *Brettanomyces spp* contenues dans un substrat liquide organique lequel contient des sucres fermentescibles, selon laquelle on prélève un échantillon dudit substrat, éventuellement on le dilue, on ajoute dans ledit substrat éventuellement dilué au moins un premier fluorochrome apte à se lier à l'ADN des cellules mortes et/ou vivantes, on irradie ledit échantillon de manière à obtenir l'émission de fluorescence dudit premier fluorochrome et on irradie ledit échantillon également de manière à obtenir une émission de fluorescence dudit échantillon à 670 nm, on établit un histogramme bi paramétrique donnant pour chaque point l'intensité de la fluorescence due au premier fluorochrome et l'intensité de la fluorescence émise à 670nm, on obtient ainsi au moins un premier nuage de points correspondant à une intensité de fluorescence émise et détectée à 670nm supérieure à celle détectée pour les autres points, on en déduit que les points dudit premier nuage correspondent aux cellules de *Brettanomyces* spp et on dénombre éventuellement le nombre de cellules de *Brettanomyces spp* en comptant les points dudit premier nuage.

C'est en effet le mérite de la Demanderesse que d'avoir mis en évidence l'autofluorescence des cellules de *Brettanomyces* spp à 670nm après excitation avec un laser émettant dans les longueurs d'ondes correspondant au rouge (de 620 nm inclus à 750 nm inclus) et en particulier à 637nm. Le premier fluorochrome se liant à l'ADN, il permet de supprimer le bruit de fond dû aux particules en suspension, par exemple. Seules les cellules des microorganismes présents, c'est-à-dire les levures, les champignons et les bactéries seront marquées. Si le premier fluorochrome ne pénètre que dans les cellules dont la paroi est altérée et donc perméable (cellules mortes), le signal de fluorescence ne permet de détecter que les cellules mortes mais la détermination de la première fenêtre comme expliqué ultérieurement et l'utilisation d'un deuxième fluorochrome se liant à la fois aux cellules mortes et aux cellules vivantes permet de détecter toutes les cellules de *Brettanomyces spp.*

La longueur d'onde d'excitation n'est pas limitée selon l'invention. Elle est avantageusement supérieure ou égale à 620 nm et inférieure ou égale à 750 nm inclus et en particulier égale à 637nm.

Par ailleurs, la mise en évidence de l'autofluorescence des cellules de *Brettanomyces spp* permet de rapidement et précisément définir une fenêtre sur les histogramme bi paramétrique, ce qui confère à la méthode de l'invention une grande précision, une grande fiabilité et une grande rapidité de mise en œuvre.

Avantageusement, le premier fluorochrome se lie à l'ADN des cellules mortes et vivantes.

L'échantillon peut être dilué au 1/10, 1/40,1/100, 1/300 ou 1/1000, en fonction de sa charge en levures et bactéries.

### Description détaillée

Selon un mode de mise en œuvre particulier, ledit substrat contient en majorité des levures *Brettanomyces spp* et des levures *Sacharomyces spp,* on obtient sur ledit histogramme bi paramétrique au moins deux nuages de points, l'un comprenant les points correspondant à une intensité de fluorescence émise à 670nm supérieure à celle des points du deuxième nuage de points, on en déduit que les points dudit premier nuage correspondent aux cellules de *Brettanomyces* spp et que les points dudit deuxième nuage correspondent aux cellules de *Saccharomyces spp* et on dénombre éventuellement le nombre de cellules de *Brettanomyces spp* et/ou de *Saccharomyces spp* en comptant les points de chacun desdits nuages.

Avantageusement, en fonction de la complexité du substrat, avant de mesurer la fluorescence, on réalise une première discrimination entre les particules et les cellules contenues dans ledit échantillon en mesurant l'intensité de la lumière réfléchie et réfractée et l'intensité de la lumière difractée, on établit un histogramme bi paramétrique donnant pour chaque point correspondant à une particule ou cellule détectée la valeur desdites intensités, on détermine ainsi en fonction des valeurs desdites intensités une première fenêtre qui contient des points affectables à des cellules de levure et éventuellement une deuxième fenêtre qui correspond à des points affectables à des cellules bactériennes, on établit ledit histogramme bi paramétrique donnant l'intensité de la fluorescence due au premier fluorochrome et l'intensité de la fluorescence émise à 670nm pour chaque point situé dans ladite première fenêtre. La détermination de la première fenêtre (stratégie de « gating ») permet de réduire le bruit de fond dû aux particules et bactéries contenues dans le substrat. On isole ainsi les points correspondant aux cellules de levures et on établit pour ces seules cellules l'histogramme bi paramétrique d'émission de fluorescence précité. Ceci permet de détecter les points qui représentent en fait soit une bactérie soit une particule et qui se trouvent encore dans la première fenêtre.

Avantageusement, ledit premier fluorochrome étant apte à se lier à l'ADN des cellules vivantes et à l'ADN des cellules dont la paroi est perméable, avant toute mesure, on ajoute en outre, audit échantillon éventuellement dilué, un deuxième fluorochrome apte à se lier uniquement à l'ADN des cellules dont la paroi est perméable, on détermine une troisième fenêtre qui entoure les points dudit premier nuage, on excite l'échantillon de manière à provoquer l'émission de fluorescence du premier et du deuxième fluorochrome et pour les points situés dans ladite troisième fenêtre, on établit également un histogramme bi paramétrique donnant pour chaque point l'intensité de la fluorescence du premier et celle du deuxième fluorochrome ou l'intensité de la fluorescence par unité de surface d'un des deux fluorochromes et celle due à l'autre fluorochrome et on détermine, en outre, deux groupes de points, un premier groupe pour lesquels la fluorescence due au fluorochrome qui se fixe uniquement à l'ADN des cellules dont la paroi est perméable est supérieure à celle du deuxième groupe et éventuellement on compte le nombre de points de chacun des groupes, lequel correspond au nombre de cellules de *Brettanomyces sppp* vivantes pour le deuxième groupe et le nombre de cellules de *Brettanomyces spp* mortes pour le premier groupe.

Il est également possible d'utiliser l'intensité de fluorescence par unité de surface pour l'un ou l'autre des fluorochromes.

Le deuxième fluorochrome permet de supprimer encore des points correspondant à des particules ou des bactéries; de tels points peuvent en effet demeurer dans la première fenêtre. Plus la longueur d'onde d'émission en fluorescence du deuxième fluorochrome est proche de 670nm, plus l'intensité de la fluorescence des cellules de *Brettanomyces spp* marquées à l'aide de ce deuxième fluorochrome est importante. Il est ainsi possible de discriminer les cellules de *Brettanomyces spp* mortes des cellules de *Brettanomyces spp* vivantes.

Il est également possible de distinguer les cellules de *Brettanomyces spp* mortes en utilisant uniquement l'auto fluorescence à 670nm. On établit alors un histogramme bi paramétrique dans la troisième fenêtre donnant la fluorescence pour le premier fluorochrome et la fluorescence détectée à 670nm. La fluorescence des cellules mortes et/ou latentes à 670nm est plus faible que celle émise par les cellules vivantes.

Avantageusement, on détermine ladite deuxième fenêtre et on excite ledit échantillon de manière à provoquer l'émission de fluorescence du premier et du deuxième fluorochrome et pour les points situés dans ladite deuxième fenêtre, on établit un histogramme bi paramétrique donnant pour chaque point l'intensité de la fluorescence du premier et celle du deuxième fluorochrome ou l'intensité de la fluorescence par unité de surface d'un des deux fluorochromes et celle due à l'autre fluorochrome et l'on détermine deux groupes de points, un premier groupe pour lesquels la fluorescence due au fluorochrome qui se fixe uniquement à l'ADN des cellules dont la paroi est perméable est supérieure à celle du deuxième groupe et on compte le nombre de points de chacun des groupes, lequel correspond au nombre de cellules bactériennes vivantes pour le deuxième groupe et le nombre de cellules bactériennes mortes pour le premier groupe.

Avec un seul et même échantillon et avec un seul passage dans le cytomètre, il est possible d'obtenir simultanément une analyse des levures et des bactéries.

De manière particulièrement avantageuse, avant toute mesure, on ajoute, en outre, audit échantillon éventuellement dilué un troisième fluorochrome qui n'émet un signal de fluorescence que lorsqu'il qu'il réagit avec une cellule vivante, on excite ledit échantillon de manière à obtenir également l'émission de fluorescence dudit troisième fluorochrome et on établit pour les points de ladite deuxième et/ou troisième fenêtre, un histogramme bi paramétrique donnant pour chaque point l'intensité de la fluorescence due au fluorochrome qui ne se lie qu'à l'ADN des cellules dont la paroi est perméable et l'intensité de la fluorescence due au troisième fluorochrome, on détermine alors pour chaque fenêtre, trois sous-groupes de points, un premier sous-groupe de points correspondants à une intensité de fluorescence due au troisième fluorochrome supérieure à celle des autres sous-groupes, ce premier sous-groupe de points représentant les cellules de *Brettanomyces* spp/cellules bactériennes vivantes et actives, un deuxième sous-groupe de points correspondant à une intensité de fluorescence due au troisième fluorochrome inférieure à celle du premier sous-groupe et couplée avec une intensité de fluorescence due au premier/deuxième fluorochrome inférieure à celle du troisième sous-groupe, les points de ce deuxième sous-groupe correspondent aux cellules de *Brettanomyces spp*/cellules bactériennes à l'état latent et un troisième sous-groupe de points correspondant à une intensité de fluorescence due au premier/deuxième fluorochrome supérieure à celle du premier et du deuxième sous-groupe, ces points correspondent aux cellules de *Brettanomyces* spp/cellules bactériennes mortes.

On voit ici que la présence des trois fluorochromes permet avec un seul échantillon analysé en un passage dans un cytomètre, non seulement de détecter simultanément la présence de levures et de bactéries, de quantifier (nombre de cellules) les levures et les bactéries, de distinguer les levures *Brettanomyces spp* des autres levures, notamment de *Saccharomyces spp* mais encore de déterminer pour les bactéries et pour les levures et plus particulièrement pour les levures *Brettanomyces spp* leur état (morte, vivante ou latente). La méthode de l'invention permet même de quantifier les levures et bactéries latentes, lesquelles ne sont pas détectables par culture. Elle s'avère également beaucoup plus rapide que la méthode de dénombrement après mise en culture.

La forme des fenêtres n'est pas limitée selon l'invention. Elles peuvent avantageusement être polygonales.

Selon l'invention les fluorochromes ne sont pas limités. Ainsi, ledit premier et ledit deuxième fluorochrome sont différents et peuvent être choisis parmi les fluorochromes aptes à se lier à l'ADN des cellules et présentant une longueur d'onde d'absorption maximale en fluorescence égale ou supérieure à 599nm et égale ou inférieure à 657nm, une longueur d'onde d'émission en fluorescence maximale égale ou supérieure à 619nm et égale ou inférieure à 678nm et un rendement quantique égal ou supérieur à 0,16 et égal ou inférieur à 0,39 et leurs mélanges, en particulier les fluorochromes apte à se lier à l'ADN des cellules et présentant une longueur d'onde d'absorption en fluorescence maximale de 652 nm, une longueur d'onde d'émission en fluorescence maximale de 676 nm et un rendement quantique de fluorescence sur l'ADN de 0,27 et les fluorochromes aptes à se lier à l'ADN et présentant une longueur d'onde d'absorption maximale en fluorescence de 657nm, une longueur d'onde d'émission maximale en fluorescence de 673nm et un rendement quantique de fluorescence sur l'ADN de 0,17, les fluorochromes aptes à se lier uniquement à l'ADN des cellules dont la paroi est perméable et qui présentent une longueur d'onde d'absorption maximale en fluorescence de 547nm, une longueur d'onde d'émission maximale en fluorescence de 570nm et un rendement quantique de fluorescence sur l'ADN de 0,9 et leur mélanges.

Lorsque ledit premier fluorochrome est choisi parmi les fluorochromes aptes à se lier à l'ADN et présentant une longueur d'onde d'absorption maximale en fluorescence de 657nm, une longueur d'onde d'émission maximale en fluorescence de 673nm et un rendement quantique de fluorescence sur l'ADN de 0,17 et les fluorochromes apte à se lier à l'ADN des cellules et présentant une longueur d'onde d'absorption en fluorescence maximale de 652 nm, une longueur d'onde d'émission en fluorescence maximale de 676 nm et un rendement quantique de fluorescence sur l'ADN de 0,27, le deuxième fluorochrome est choisi parmi les fluorochromes aptes à se lier uniquement à l'ADN des cellules dont la paroi est perméable et qui présentent une longueur d'onde d'absorption maximale en fluorescence de 547nm et une longueur d'onde d'émission maximale en fluorescence de 570nm et un rendement quantique de fluorescence sur l'ADN de 0,9. Ledit troisième fluorochrome est avantageusement choisi parmi le diacétate de 5-carboxyfluoresceine, le diacétate de 6-carboxyfluoresceine, les mélanges de diacétate de 5-carboxyfluoresceine et de diacétate de 6-carboxyfluoresceine et le diacétate fluorescéine 5,6 carboxylate de succinimidyl de formule générale (1) suivante :

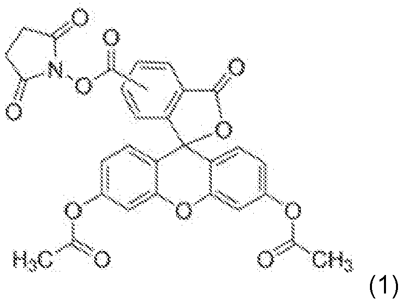

Ainsi, selon un mode de mise en œuvre particulier, ledit premier et ledit deuxième fluorochrome peut être choisi parmi les fluorochromes commercialisés par la société Thermofisher sous les appellations SYTOX-orange^{®}, SYTO 62^{®} et SYTO 63^{®}. Lorsque ledit premier fluorochrome est choisi parmi SYTO 62^{®} et SYTO(63), le deuxième fluorochrome est le SYTOX-orange^{®}. Inversement, lorsque le deuxième fluorochrome est le SYTOX-orange, le deuxième fluorochrome est choisi parmi SYTO 62^{®} et SYTO 63^{®} et leurs mélanges.

Le fluorochrome SYTOX Orange^{®} ne pénètre que dans les cellules dont la paroi est altérée, c'est-à-dire les cellules mortes. Il est de préférence utilisé en combinaison avec le troisième fluorochrome pour discriminer les différents états des cellules de *Brettanomyces spp* (vivant, latent ou mort)

Le fluorochrome SYTOX-orange ^{®} est commercialisé par la société Thermofisher. Il est apte à se lier à l'ADN, présente une longueur d'onde d'absorption maximale en fluorescence de 547nm et une longueur d'onde d'émission maximale en fluorescence de 570nm et un rendement quantique de fluorescence sur l'ADN de 0,9.

Un fluorochrome apte à se lier à l'ADN des cellules et présentant une longueur d'onde d'absorption en fluorescence maximale de 652 nm et une longueur d'onde d'émission en fluorescence maximale de 676 nm et un rendement quantique de fluorescence sur l'ADN de 0,27 est commercialisé sous l'appellation SYTO 62^{®} par la société Thermofisher.

Un fluorochrome apte à se lier à l'ADN et présentant une longueur d'onde d'absorption maximale en fluorescence de 657nm et une longueur d'onde d'émission maximale en fluorescence de 673nm et un rendement quantique de fluorescence sur l'ADN de 0,17 est commercialisé sous l'appellation SYTO 63^{®} par la société Thermofisher.

Le substrat n'est pas limité selon l'invention. Ainsi, le substrat peut être choisi parmi le vin pétillant ou non, le vin rouge, le vin blanc, le vin rosé, le cidre, la bière, le saké, les jus de fruits, notamment de raisin ou de pomme, le kéfir d'eau, le kéfir de jus de fruit, le kéfir de lait, le lait, la tequila, le whisky, la vodka, les moûts notamment de raisin, les vins en cours de première ou de deuxième fermentation, les vins finis, pétillants ou non et les vinaigres.

La méthode de l'invention peut s'appliquer à toute espèce de levure *Brettanomyces* spp choisie parmi les espèces suivantes *B*. *anomalus, B. bruxellensis, B. custersianus, B. nanus, B. dekkera bruxellensis et B. naardenensis* d'au moins une autres espèce de levure et notamment

Elle permet notamment de discriminer les cellules de *Brettanomyces spp* des cellules d'au moins une espèce de *Saccharomyces spp* choisie parmi les espèces suivantes : *Saccharomyces bailii Linder, Saccharomyces carlsbergensis, Saccharomyces cerevisiae, Saccharomyces delbrueckii, Saccharomyces exiguus, Saccharomyces fermentati, Saccharomyces florentinus, Saccharomyces fragilis, Saccharomyces fructuum, Saccharomyces heterogenicus, Saccharomyces oleaginosus, Saccharomyces rosei, Saccharomyces steineri, Saccharomyces boulardii, Saccharomyces kefir, Saccharomyces kluyveri.*

La méthode de l'invention permet en particulier de discriminer Brettanomyces dekkera bruxellensis ou Brettanomyces bruxellensis de Saccharomyces cerevisiae. La présente invention concerne également un mélange fluorochromique contenant ou constitué d'un solvant et d'un premier fluorochrome choisi parmi les fluorochromes aptes à se lier à l'ADN et présentant une longueur d'onde d'absorption en fluorescence maximale de 652 nm et une longueur d'onde d'émission en fluorescence maximale de 676 nm et un rendement quantique de fluorescence sur l'ADN de 0,27, les fluorochromes aptes à se lier à l'ADN et présentant une longueur d'onde d'absorption maximale en fluorescence de 657nm et une longueur d'onde d'émission maximale en fluorescence de 673nm et un rendement quantique de fluorescence sur l'ADN de 0,17 et leur mélanges, un deuxième fluorochrome choisi parmi les fluorochromes présentant une longueur d'onde d'absorption maximale en fluorescence de 547nm et une longueur d'onde d'émission maximale en fluorescence de 570nm et un rendement quantique de fluorescence sur l'ADN de 0,9 et un troisième fluorochrome ledit troisième fluorochrome est choisi parmi le diacétate de 5-carboxyfluoresceine, le diacétate de 6-carboxyfluoresceine, les mélanges de diacétate de 5-carboxyfluoresceine et de diacétate de 6-carboxyfluoresceine et le diacétate fluorescéine 5,6 carboxylate de succinimidyl de formule générale (1) suivante :

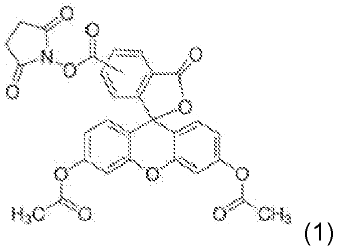

La présente invention concerne également le mélange fluorochromique précité pour son utilisation dans la détection *in vitro* (plus particulièrement dans un substrat tel que précité) de *Brettamyces spp* et sa discrimination d'avec les autres levures, en particulier *Saccharomyces spp.*

La présente invention concerne également l'utilisation du mélange fluorochromique précité pour la détection et la discrimination de *Brettamyces spp* et sa discrimination d'avec les autres levures, en particulier *Saccharomyces spp,* notamment dans un substrat tel que précité.

Selon un mode de réalisation particulier, le mélange fluorochromique contient le SYTOX^{®}-orange, le SYTO^{®}62 ou le SYTO^{®} 63 et le mélange c-FDA. De la même manière, selon un mode de réalisation avantageux, la méthode de l'invention met en œuvre ce mélange précité de trois fluorochromes.

Le mélange est ajouté dans le substrat éventuellement dilué avant son analyse dans le cytomètre en flux.

### Définitions

L'acronyme FSC fait référence au signal correspondant à la lumière diffractée (FSC: Forward Scatter) ; ce signal dépend de la taille et de la surface de la cellule analysée ;
L'acronyme SSC (SSC : Side Scatter) fait référence au signal correspondant à la lumière réfléchie et réfractée ; ce signal dépend de la granularité et de la complexité cellulaire de la cellule analysée.

L'acronyme SSC-H fait référence à l'intensité du signal correspondant à la lumière réfléchie et réfractée ;
L'acronyme SSC-A fait référence à l'intensité par unité de surface du signal correspondant à la lumière réfléchie et réfractée ;
Les acronymes FSC-H et FSC-A font référence, respectivement à l'intensité et à l'intensité par unité de surface du signal correspondant à la lumière difractée ;
L'acronyme cFDA ou c-FDA désigne un mélange de diacétate de 5-carboxy fluorescéine et de diacétate de 6-carboxy fluorescéine.

Dans toute la demande, les valeurs maximales de fluorescence en absorption et en émission définissant les fluorochromes sont déterminées en présence d'ADN avec un ratio d'environ 100 paires de bases et notamment 100 paires bases d'acide nucléique pour une molécule de fluorochrome dans un milieu Tris de pH 7,5 et de concentration en EDTA égale à 1mM.

Dans toute la demande, le rendement quantique de fluorescence qui définit les fluorochromes est mesuré en présence d'ADN et exprimé de manière relative au rendement déterminé pour le violet de crésyle dans le méthanol.

Les termes « en majorité » signifient 50% ou plus en masse ou en nombre. Lorsqu'ils s'appliquent à deux entités, ils signifient que le mélange des deux entités est présent à 50% ou plus en masse ou en nombre.

Le terme « jus» désigne au sens de la présente invention tout liquide extrait de la pulpe, de la chair de certains fruits ou légumes.

Le terme « moût » désigne au sens de la présente invention une mixture obtenue par pressurage ou cuisson de végétaux (graines, fruits, feuilles...) ou bien d'extraits de végétaux. Ces végétaux peuvent être des fruits, des légumes comme la pomme de terre mais aussi des céréales comme le blé, l'orge, le malt, le maïs ou le riz, par exemple.

Le terme « vin » désigne au sens de la présente invention un jus ou un moût de raisin blanc et/ou noir dont une partie ou la totalité du sucre est transformée en alcool par fermentation, notamment par la fermentation alcoolique due à *Saccharomyces cerevisiae.* Le vin peut être blanc, rouge ou rosé, selon l'invention. Le vin peut être conservé dans un tonneau de bois.

Le terme « vin fini » désigne un vin, rouge, blanc, rosé, pétillant ou non, dont la fermentation est terminée (première et deuxième dans le cas des vins pétillants) et qui sont stockées en cuve, en tonneau de bois ou en bouteille.

Le terme « bière » désigne tout boisson alcoolisée obtenue par fermentation d'une levure ou d'un champignon.

Le terme « vinaigre» désigne au sens de la présente invention le résultat de la fermentation acétique produite par oxydoréduction microbiologique d'une solution aqueuse d'éthanol, notamment un vin ou un cidre, exposée à l'air.

### FIGURES

La Fig. 1a est un histogramme bi paramétrique représentant l'intensité du signal SSC en fonction de l'intensité du signal FSC obtenu pour l'analyse d'un échantillon de vin fini ;
La Fig. 1b est un histogramme bi paramétrique représentant l'intensité de la fluorescence due au fluorochrome SYTOX-orange^{®} en fonction de l'intensité de la fluorescence due au fluorochrome SYTO 62^{®} pour les points situés dans la fenêtre "Yeast and Bacteria background" (première fenêtre) représentée sur la Fig. 1a ;
La Fig. 1c est un histogramme bi paramétrique représentant l'intensité de la fluorescence due au fluorochrome cFDA en fonction de la fluorescence due au fluorochrome SYTOX-orange^{®} obtenu pour la fenêtre « Brettanomyces » (deuxième fenêtre) visible sur la Fig. 1b ;
La Fig. 1d est un histogramme bi paramétrique donnant l'intensité de la fluorescence détectée dans le canal RL1 à une longueur d'onde de 670 nm et l'intensité de la florescence détectée dans le canal GL1 à 575 nm ;
La Fig. 2 représente un histogramme bi paramétrique donnant pour chaque point l'intensité du signal SSC en fonction de l'intensité du signal FSC, la deuxième fenêtre qui correspond aux bactéries est visible sur cette figure ;
La Fig. 3 représente un histogramme bi paramétrique donnant l'intensité par unité de surface du signal SSC en fonction de l'intensité de la fluorescence du fluorochrome SYTO-62^{®} pour les points situés dans la fenêtre "bacteria + background » (deuxième fenêtre) visible sur la Fig. 2; la fenêtre visible sur la Fig. 3 entoure les points correspondants à cellules bactériennes vivantes ;
La Fig. 4 représente l'intensité de la fluorescence émise par le fluorochrome SYTOX-orange^{®} en fonction de l'intensité de fluorescence due au mélange cFDA pour les points situés dans la deuxième fenêtre, elle représente les trois groupes de points qui correspondent aux trois état des cellules bactériennes (vivante, latente et morte).

### EXEMPLES :

### Mélange de marquage fluorochromique

De l'eau physiologique (eau osmosée/ultra pure + NaCl à 7g/L) est préparée puis autoclavée et filtrée avant utilisation (seuil de coupure du filtre 0.22µm).

Un fluorochrome commercialisé sous l'appellation SYTO 62^{™} (le SYTO 63^{™} est également utilisable) (Thermofischer, 5µM), le SYTOX^{™}-Orange (Thermofischer, 5µM) et le mélange de diacétate de 5,6 carboxy fluorescéine (c-FDA) sont diluées dans du DMSO (respectivement 50µM, 12,5µM et 2g/L en concentrations finales) puis stockées au congélateur. Les concentrations finales de fluorochromes dans le mélange de marquage sont de 0,15µM de SYTO 62^{™}ou 63^{™}, 0,025µM de SYTOX^{™}-Orange (SYTOX-or) et 2mg/L de c-FDA dans de l'eau physiologique.

Le premier fluorochrome présente à forte affinité pour l'ADN et fait fluorescer les organismes biologiques contenant de l'ADN. Il permet de séparer les cellules microbiologiques du bruit de fond du vin. Le fluorochrome choisi ici est le SYTO^{®}-63. Sa fluorescence est induite par le laser rouge (637 nm). Des résultats similaires sont également obtenus dans les mêmes conditions avec le SYTO^{®} 62.

Le deuxième fluorochrome perméant ne pénètre que dans les cellules dont la paroi est compromise. Le but est de séparer les cellules aux membranes perméables (marquées positivement) correspondant théoriquement aux cellules mortes, des cellules vivantes (non marquées). Le fluorochrome choisi ici est le SYTOX^{®}-Orange dont la fluorescence est induite par le laser vert (532 nm).

Le troisième fluorochrome est un fluorochrome inactif sous sa forme initiale d'ester. Il devient actif par l'activité estérase des métabolismes cellulaire. L'objectif est de séparer les cellules métaboliquement actives de celles qui ne le sont pas. Cette deuxième catégorie sont les populations sous formes latentes qui, en pratique, ne se développent peu ou pas dans les cultures sur boîte de pétri. Elle correspond factuellement aux populations VNC (populations viables non cultivables). Le fluorochrome choisi ici est le c-FDA. Sa fluorescence est induite par le laser bleu (488 nm).

### Préparation des échantillons de substrat

### Échantillons de vins et moûts

Les échantillons de vins finis ou en cours de fermentation (moût) sont dilués (au 1/40,1/100, 1/300 ou 1/1000 en fonction de leur charge biologique) avec le mélange fluorochromique de marquage. Pour les vins conditionnés, 50mL sont centrifugés 8 min à 4500rpm. Le surnageant est éliminé et le culot est repris dans 10mL d'eau physiologique filtrée (8,5g/L de NaCl dans de l'eau osmosée et filtrée à 0,22µm). Une dilution de l'échantillon au ½ dans du mélange de marquage est réalisée puis l'ensemble est vortexé quelques secondes. Le mélange substrat+mélange fluorochromique incube environ 30 min à l'obscurité avant analyse.

L' échantillon est un échantillon de vin fini de 2020 de la région Languedoc.

### Matériel utilisé

Cytomètre : ATTUNE^{®} NXT acoustic focusing cytometer (thermofischer scientific). La présente méthode décrit un protocole d'analyse microbiologique employant un cytomètre en flux doté de 3 lasers: bleu (488 nm), vert (532 nm) et rouge (637 nm). Un triple marquage cellulaire des bactéries et levures à partir des fluorochromes précités est réalisé.

Le cytomètre est muni, ou non, d'un passeur automatique pour la lecture de microplaques 96 puits. Cet équipement est doté d'un système de focalisation acoustique du flux permettant d'utiliser un débit jusqu'à 1000 µL/min.

Le débit du flux est fixé à 500 µL/min. Ce dernier est ralenti lorsque la charge microbienne est importante. Les données sont récoltées sur les canaux FSC, SSC, BL1 (525/50) pour c-FDA, GL1 (575/36) pour SYTOX-orange et RL1 (670/14) pour SYTO 62 ou 63. Les valeurs des voltages pour chacun de ces canaux sont respectivement 200V, 300V, 330V, 360V et 440V. Des valeurs différentes peuvent être appliquées. Il n'existe pas de problème de compensation à corriger dans cette configuration.

### Traitement des résultats

Sont considérés comme microorganismes, les évènements répondant au SYTO 6255^{®} ou 63 positif (SYTO 62 ou 63 +).

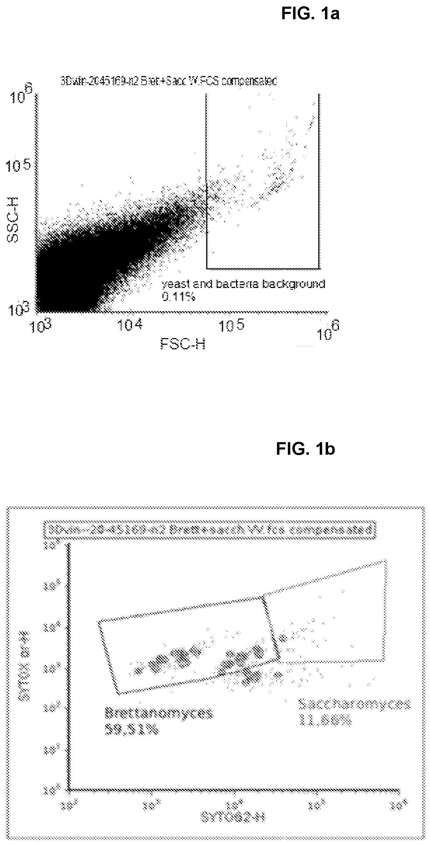

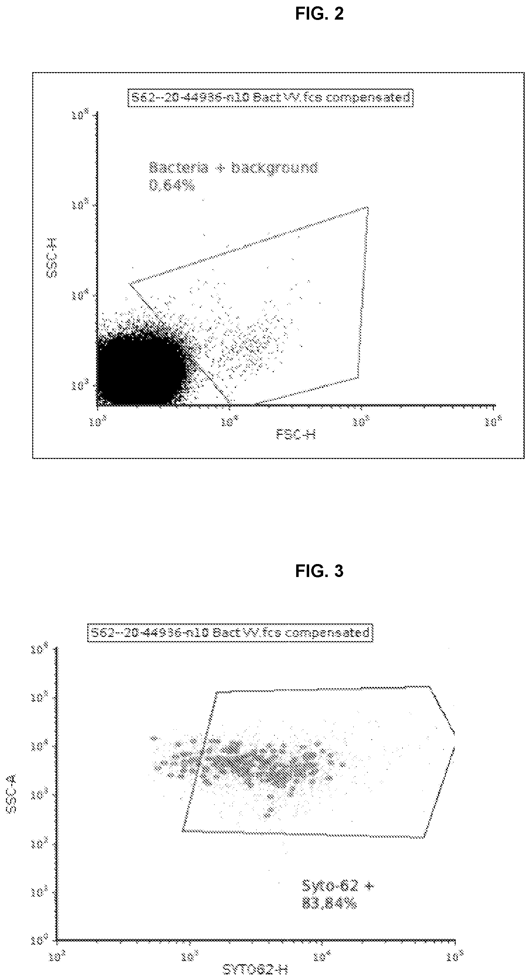

Le triple marquage des échantillons et une stratégie de d'étalonnage ont permis la séparation des bactéries, des levures *Saccharomyces* et *Brettanomyces* dans le vin fini. Dans un premier temps, la majorité des bactéries et le bruit de fond sont séparés des levures et quelques bactéries par le plot SSC-H/FSC-H (Fig. 1a et 2). Ensuite, grâce au plot RL1 (670/14 nm) appliqué sur la fenêtre levure-bactéries de la Fig. 1a, les dernières bactéries sont éliminées et la dichotomie entre les *Brettanomyces* et *Saccharomyces* apparaît. Cette dichotomie est rendue possible grâce à une propriété manifeste de niveau relatifs différents d'autofluorescence dans le rouge des cellules de *Saccharomyces spp* et celles de *Brettanomyces spp.*

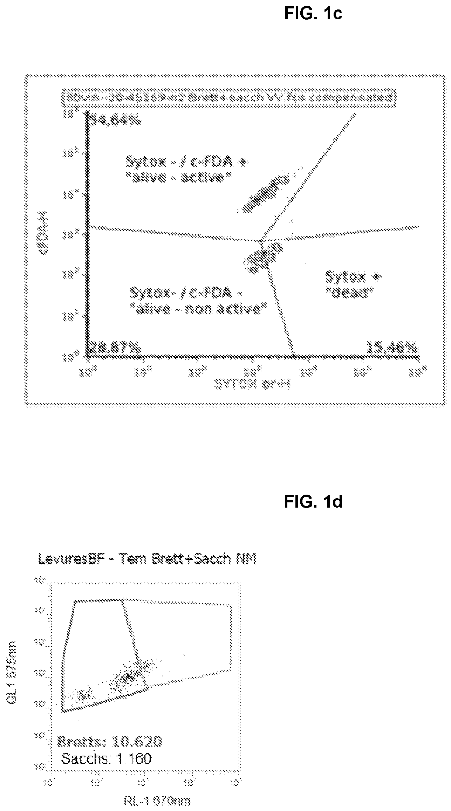

Comme représenté sur la Fig. 1d, même en l'absence de marquage SYTO62/63, la différence de fluorescence à 670nm entre *Saccharomyces* et *Brettanomyces* est observable (voir Fig. 1d). Il s'agit donc bien d'un phénomène différencié d'autofluorescence.

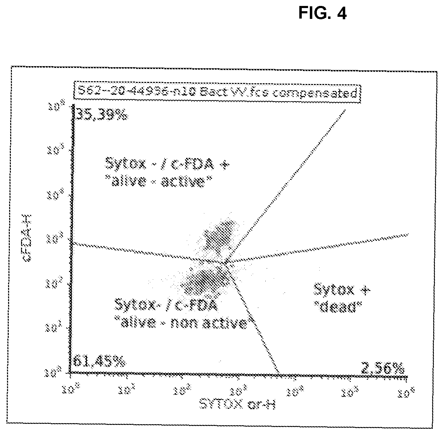

La Fig. 3 montre la différence de fluorescence entre les cellules de bactéries mortes et celles latentes ou vivantes. La Fig. 1b montre cette différence pour les bactéries. Ensuite, les différents états des *Brettanomyces* et des bactéries sont obtenus par croisement des données obtenues par les 3 fluorochromes. Ainsi l'état "mort" correspond au SYTOX-Orange + / cFDA -, l'état "vivant latent (VNC)" au SYTOX-Orange - / c-FDA -, et l'état "vivant actif/vitale" au SYTOX-Orange - / c-FDA + (voir Fig. 1c et Fig. 4). Les fenêtres ont pu être validées par le travail de comparaison de la cytométrie avec la microscopie et les boîtes de Pétri.

On constate au vu des résultats précites que la méthode de l'invention permet de réaliser en même temps, une séparation du microorganisme d'intérêt du bruit de fond, une séparation des microorganismes vivants des microorganismes mort et à l'intérieur de la population des microorganismes vivants, séparer les microorganismes physiologiquement actifs, des microorganismes en dormance.

Elle permet d'apporter toutes les informations sur la vitalité et la viabilité en même temps. Elle peut être mise en œuvre rapidement avec un marquage actif en 15 minutes environ. Elle est de plus peu couteuse car elle utilise des réactifs existants. Elle est industrialisable pour de l'analyse à haut débit.

Naturellement, l'invention est décrite dans ce qui précède à titre d'exemple. Il est entendu que l'homme du métier est à même de réaliser différentes variantes de réalisation de l'invention sans pour autant sortir du cadre de l'invention.

## Revendications

1. Méthode de détection, de quantification et de discrimination par cytométrie en flux des cellules des levures *Brettanomyces spp* contenues dans un substrat liquide organique lequel contient des sucres fermentescibles, selon laquelle on prélève un échantillon dudit substrat, éventuellement on le dilue, on ajoute dans ledit substrat éventuellement dilué au moins un premier fluorochrome apte à se lier à l'ADN des cellules mortes et/ou vivantes, on irradie ledit échantillon de manière à obtenir l'émission de fluorescence dudit premier fluorochrome et on irradie ledit échantillon également de manière à obtenir une émission de fluorescence dudit échantillon à 670 nm, on établit un histogramme bi paramétrique donnant pour chaque point l'intensité de la fluorescence due au premier fluorochrome et l'intensité de la fluorescence émise à 670nm, on obtient ainsi au moins un premier nuage de points correspondant à une intensité de fluorescence émise et détectée à 670nm supérieure à celle détectée pour les autres points, on en déduit que les points dudit premier nuage correspondent aux cellules de *Brettanomyces* spp et on dénombre éventuellement le nombre de cellules de *Brettanomyces spp* en comptant les points dudit premier nuage.

2. Méthode selon la revendication 1, **caractérisée en ce que** ledit substrat contient en majorité des levures *Brettanomyces spp* et des levures *Sacharomyces spp,* **en ce que** l'on obtient sur ledit histogramme bi paramétrique deux nuages de points, un premier nuage comprenant les points correspondant à une intensité de fluorescence émise à 670nm supérieure à celle des points du deuxième nuage de points, **en ce que** l'on en déduit que les points dudit premier nuage correspondent aux cellules de *Brettanomyces* spp et que les points dudit deuxième nuage correspondent aux cellules de *Saccharomyces spp* et on dénombre éventuellement le nombre de cellules de *Brettanomyces spp* et/ou de Saccharomyces spp en comptant les points de chacun desdits nuages.

3. Méthode selon la revendication 1 ou 2, **caractérisée en ce qu'**avant de mesurer la fluorescence, on réalise une première discrimination entre les particules et les cellules contenues dans ledit échantillon en mesurant l'intensité de la lumière réfléchie et réfractée et l'intensité de la lumière difractée, on établit un histogramme bi paramétrique donnant pour chaque point correspondant à une particule ou cellule détectée la valeur desdites intensités, on détermine ainsi en fonction des valeurs desdites intensités une première fenêtre qui contient des points affectables à des cellules de levure et éventuellement une deuxième fenêtre qui correspond à des points affectables à des cellules bactériennes, on établit ledit histogramme bi paramétrique donnant l'intensité de la fluorescence due au premier fluorochrome et l'intensité de la fluorescence émise à 670nm pour chaque point situé dans ladite première fenêtre.

4. Méthode selon l'une quelconque des revendications précédentes, **caractérisée en ce que** ledit premier fluorochrome étant apte à se lier à l'ADN des cellules vivantes et à l'ADN des cellules dont la paroi est perméable, **en ce qu'**avant toute mesure, on ajoute en outre, audit échantillon éventuellement dilué, un deuxième fluorochrome apte à se lier uniquement à l'ADN des cellules dont la paroi est perméable , **en ce que** l'on détermine en outre, une troisième fenêtre qui entoure les points dudit premier nuage, **en ce qu'**on excite l'échantillon de manière à provoquer l'émission de fluorescence du premier et du deuxième fluorochrome et pour les points situés dans ladite troisième fenêtre, on établit également un histogramme bi paramétrique donnant pour chaque point l'intensité de la fluorescence du premier et celle du deuxième fluorochrome ou l'intensité de la fluorescence par unité de surface d'un des deux fluorochromes et celle due à l'autre fluorochrome et **en ce que** l'on détermine en outre, deux groupes de points, un premier groupe pour lesquels la fluorescence due au fluorochrome qui se fixe uniquement à l'ADN des cellules dont la paroi est perméable est supérieure à celle du deuxième groupe et on compte le nombre de points de chacun des groupes, lequel correspond au nombre de cellules de *Brettanomyces sppp* vivantes pour le deuxième groupe et le nombre de cellules de *Brettanomyces spp* mortes pour le premier groupe.

5. Méthode selon les revendications 2 et 3, **caractérisée en ce que** l'on détermine ladite deuxième fenêtre et **en ce qu'**on excite également ledit échantillon de manière à provoquer l'émission de fluorescence du premier et du deuxième fluorochrome et pour les points situés dans ladite deuxième fenêtre, on établit un histogramme bi paramétrique donnant pour chaque point l'intensité de la fluorescence du premier et celle du deuxième fluorochrome ou l'intensité de la fluorescence par unité de surface d'un des deux fluorochromes et celle due à l'autre fluorochrome et **en ce que** l'on détermine deux groupes de points, un premier groupe pour lesquels la fluorescence due au fluorochrome qui se fixe uniquement à l'ADN des cellules dont la paroi est perméable est supérieure à celle du deuxième groupe et on compte le nombre de points de chacun des groupes, lequel correspond au nombre de cellules bactériennes vivantes pour le deuxième groupe et le nombre de cellules bactériennes mortes pour le premier groupe.

6. Méthode selon la revendication 5 ou 6, **caractérisée en ce que** avant toute mesure, l'on ajoute, en outre, audit échantillon éventuellement dilué un troisième fluorochrome qui n'émet un signal de fluorescence que lorsqu'il qu'il réagit avec une cellule vivante, on excite ledit échantillon de manière à obtenir également l'émission de fluorescence dudit troisième fluorochrome et on établit pour les points de ladite deuxième et/ou troisième fenêtre, un histogramme bi paramétrique donnant pour chaque point l'intensité de la fluorescence due au fluorochrome qui ne se lie qu'à l'ADN des cellules dont la paroi est perméable et l'intensité de la fluorescence due au troisième fluorochrome, on détermine alors pour chaque fenêtre, trois sous-groupes de points, un premier sous-groupe de points correspondants à une intensité de fluorescence due au troisième fluorochrome supérieure à celle des autres sous-groupes, ce premier sous-groupe de points représentant les cellules de *Brettanomyces* spp/cellules bactériennes vivantes et actives, un deuxième sous-groupe de points correspondant à une intensité de fluorescence due au troisième fluorochrome inférieure à celle du premier sous-groupe et couplée avec une intensité de fluorescence due au premier/deuxième fluorochrome inférieure à celle du troisième sous-groupe, les points de ce deuxième sous-groupe correspondent aux cellules de *Brettanomyces spp*/cellules bactériennes à l'état latent et un troisième sous-groupe de points correspondant à une intensité de fluorescence due au premier/deuxième fluorochrome supérieure à celle du premier et du deuxième sous-groupe, ces points correspondent aux cellules de *Brettanomyces spp*/cellules bactériennes mortes.

7. Méthode selon l'une quelconque des revendications précédentes, **caractérisée en ce que** ledit premier et ledit deuxième fluorochrome sont différents et choisis parmi les fluorochromes aptes à se lier à l'ADN des cellules et présentant une longueur d'onde d'absorption maximale en fluorescence égale ou supérieure à 599nm et égale ou inférieure à 657nm, une longueur d'onde d'émission en fluorescence maximale égale ou supérieure à 619nm et égale ou inférieure à 678nm et un rendement quantique égal ou supérieur à 0,16 et égal ou inférieur à 0,39 et leurs mélanges, en particulier les fluorochromes apte à se lier à l'ADN des cellules et présentant une longueur d'onde d'absorption en fluorescence maximale de 652 nm, une longueur d'onde d'émission en fluorescence maximale de 676 nm et un rendement quantique de fluorescence sur l'ADN de 0,27 et les fluorochromes aptes à se lier à l'ADN et présentant une longueur d'onde d'absorption maximale en fluorescence de 657nm, une longueur d'onde d'émission maximale en fluorescence de 673nm et un rendement quantique de fluorescence sur l'ADN de 0,17, les fluorochromes aptes à se lier uniquement à l'ADN des cellules dont la paroi est perméable et qui présentent une longueur d'onde d'absorption maximale en fluorescence de 547nm, une longueur d'onde d'émission maximale en fluorescence de 570nm et un rendement quantique de fluorescence sur l'ADN de 0,9 et leur mélanges et **en ce que** lorsque ledit premier fluorochrome est choisi parmi les fluorochromes aptes à se lier à l'ADN et présentant une longueur d'onde d'absorption maximale en fluorescence de 657nm, une longueur d'onde d'émission maximale en fluorescence de 673nm et un rendement quantique de fluorescence sur l'ADN de 0,17 et les fluorochromes apte à se lier à l'ADN des cellules et présentant une longueur d'onde d'absorption en fluorescence maximale de 652 nm, une longueur d'onde d'émission en fluorescence maximale de 676 nm et un rendement quantique de fluorescence sur l'ADN de 0,27, le deuxième fluorochrome est choisi parmi les fluorochromes aptes à se lier uniquement à l'ADN des cellules dont la paroi est perméable et qui présentent une longueur d'onde d'absorption maximale en fluorescence de 547nm et une longueur d'onde d'émission maximale en fluorescence de 570nm et un rendement quantique de fluorescence sur l'ADN de 0,9 et **en ce que** ledit troisième fluorochrome est choisi parmi le diacétate de 5-carboxyfluoresceine, le diacétate de 6-carboxyfluoresceine, les mélanges de diacétate de 5-carboxyfluoresceine et de diacétate de 6-carboxyfluoresceine et le diacétate fluorescéine 5,6 carboxylate de succinimidyl de formule générale (1) suivante :

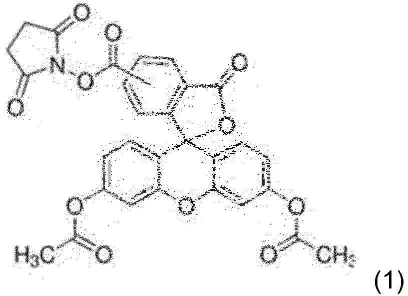

8. Méthode selon l'une quelconque des revendications précédentes, **caractérisée en ce que** ledit substrat est choisi parmi le vin pétillant ou non, le vin rouge, le vin blanc, le vin rosé, le cidre, la bière, le saké, les jus de fruits, notamment de raisin ou de pomme, le kéfir d'eau, le kéfir de jus de fruit, le kéfir de lait, le lait, la tequila, le whisky, la vodka, les moûts notamment de raisin, les vins en cours de première ou de deuxième fermentation, les vins finis, pétillants ou non et les vinaigres.

9. Méthode selon l'une quelconque des revendications précédentes, **caractérisée en ce qu'**elle permet de détecter quantifier et discriminer au moins une espèce de levure *Brettanomyces* spp choisie parmi les espèces suivantes *B. anomalus, B. bruxellensis, B. custersianus, B. nanus, B. dekkera bruxellensis et B. naardenensis* d'au moins une autres espèce de levure et notamment d'au moins une espèce de *Saccharomyces spp* choisie parmi les espèces suivantes : *Saccharomyces bailii Linder, Saccharomyces carlsbergensis, Saccharomyces cerevisiae, Saccharomyces delbrueckii, Saccharomyces exiguus, Saccharomyces fermentati, Saccharomyces florentinus, Saccharomyces fragilis, Saccharomyces fructuum, Saccharomyces heterogenicus, Saccharomyces oleaginosus, Saccharomyces rosei, Saccharomyces steineri, Saccharomyces boulardii, Saccharomyces kefir, Saccharomyces kluyveri* et en particulier *Saccharomyces cerevisiae.*

10. Méthode selon l'une quelconque des revendications précédentes, **caractérisée en ce que** l'on excite ledit échantillon à une longueur d'onde supérieure ou égale à 620 nm et inférieure ou égale à 750 nm inclus et en particulier égale à 637nm.

## Patentansprüche

1. Verfahren zum Nachweisen, Quantifizieren und Unterscheiden von *Brettanomyces spp*.-Hefezellen, die in einem organischen flüssigen Substrat enthalten sind, das fermentierbare Zucker enthält, mittels Durchflusszytometrie, wobei eine Probe des Substrats entnommen wird, diese gegebenenfalls verdünnt wird, zu dem gegebenenfalls verdünnten Substrat mindestens ein erstes Fluorochrom hinzugefügt wird, das geeignet ist, sich an die DNA toter und/oder lebender Zellen zu binden, die Probe bestrahlt wird, um die Fluoreszenzemission des ersten Fluorochroms zu erhalten, und die Probe ebenfalls bestrahlt wird, um auch eine Fluoreszenzemission der Probe bei 670 nm zu erhalten, ein biparametrisches Histogramm erstellt wird, das für jeden Punkt die Intensität der durch das erste Fluorochrom verursachten Fluoreszenz und die Intensität der bei 670 nm emittierten Fluoreszenz angibt, mindestens eine erste Punktwolke dadurch erhalten wird, die einer bei 670 nm emittierten und nachgewiesenen Fluoreszenzintensität entspricht, die höher ist als die für die anderen Punkte nachgewiesene, woraus abgeleitet wird, dass die Punkte der ersten Punktwolke den Zellen von *Brettanomyces* spp. entsprechen, und gegebenenfalls die Anzahl der Zellen von *Brettanomyces spp.* gezählt wird, indem die Punkte der ersten Punktwolke gezählt werden.

2. Verfahren nach Anspruch 1, **dadurch gekennzeichnet, dass** das Substrat überwiegend *Brettanomyces* spp.-Hefen und Sacharomyces spp.-Hefen enthält, dass auf dem biparametrischen Histogramm zwei Punktwolken erhalten werden, wobei eine erste Punktwolke die Punkte umfasst, die einer bei 670 nm emittierten Fluoreszenzintensität entsprechen, die höher ist als die der Punkte der zweiten Punktwolke, dass daraus abgeleitet wird, dass die Punkte der ersten Wolke den Zellen von *Brettanomyces spp.* entsprechen und dass die Punkte der zweiten Wolke den Zellen von *Saccharomyces spp.* entsprechen, und dass gegebenenfalls die Anzahl der Zellen von *Brettanomyces spp.* und/oder Saccharomyces spp. gezählt wird, indem die Punkte jeder dieser Wolken gezählt werden.

3. Verfahren nach Anspruch 1 oder 2, **dadurch gekennzeichnet, dass** vor der Messung der Fluoreszenz eine erste Unterscheidung zwischen den Partikeln und den in der Probe enthaltenen Zellen durchgeführt wird, indem die Intensität des reflektierten und gebrochenen Lichts und der Intensität des gebrochenen Lichts gemessen werden, ein biparametrisches Histogramm erstellt wird, das für jeden Punkt, der einem nachgewiesenen Partikel oder einer nachgewiesenen Zelle entspricht, den Wert der Intensitäten angibt, dadurch in Abhängigkeit von den Werten der Intensitäten ein erstes Fenster bestimmt wird, das Punkte enthält, die Hefezellen zuzuordnen sind, und gegebenenfalls ein zweites Fenster, das Punkten entspricht, die Bakterienzellen zuzuordnen sind, das biparametrische Histogramm erstellt wird, das die Intensität der Fluoreszenz aufgrund des ersten Fluorochroms und die bei 670 nm emittierte Intensität der Fluoreszenz für jeden Punkt in dem ersten Fenster angibt.

4. Verfahren nach einem der vorhergehenden Ansprüche, **dadurch gekennzeichnet, dass** das erste Fluorochrom geeignet ist, sich an die DNA lebender Zellen und an die DNA von Zellen mit durchlässiger Wand zu binden, dass vor jeder Messung ferner ein zweites Fluorochrom, das geeignet ist, sich nur an die DNA von Zellen mit durchlässiger Wand zu binden, zu der gegebenenfalls verdünnten Probe hinzugefügt wird, dass ferner ein drittes Fenster bestimmt wird, das die Punkte der ersten Wolke umgibt, dass die Probe so angeregt wird, dass die Fluoreszenzemission des ersten und des zweiten Fluorochroms induziert wird, und dass für die Punkte, die sich in dem dritten Fenster befinden, auch ein biparametrisches Histogramm erstellt wird, das für jeden Punkt die Intensität der Fluoreszenz des ersten und des zweiten Fluorochroms oder die Intensität der Fluoreszenz pro Flächeneinheit eines der beiden Fluorochrome und diejenige aufgrund des anderen Fluorochroms angibt, und dass ferner zwei Punktgruppen bestimmt werden, wobei in einer ersten Gruppe die Fluoreszenz aufgrund des Fluorochroms, das sich nur an die DNA von Zellen mit durchlässiger Wand bindet, höher ist als die der zweiten Gruppe, und die Anzahl der Punkte jeder der Gruppen gezählt wird, die der Anzahl lebender *Brettanomyces spp*.-Zellen für die zweite Gruppe und der Anzahl toter *Brettanomyces* spp.-Zellen für die erste Gruppe entspricht.

5. Verfahren nach den Ansprüchen 2 und 3, **dadurch gekennzeichnet, dass** das zweite Fenster bestimmt wird und dass die Probe ebenfalls so angeregt wird, dass die Fluoreszenzemission des ersten und des zweiten Fluorochroms induziert wird, und dass für die Punkte, die sich innerhalb des zweiten Fensters befinden, ein biparametrisches Histogramm erstellt wird, das für jeden Punkt die Fluoreszenzintensität des ersten und des zweiten Fluorochroms oder die Fluoreszenzintensität pro Flächeneinheit eines der beiden Fluorochrome und die Intensität des anderen Fluorochroms angibt, und dass zwei Punktgruppen bestimmt werden, wobei in einer ersten Gruppe die Fluoreszenz aufgrund des Fluorochroms, das sich nur an die DNA von Zellen mit durchlässiger Wand bindet, höher ist als die der zweiten Gruppe, und die Anzahl der Punkte jeder der Gruppen gezählt wird, die der Anzahl lebender Bakterienzellen für die zweite Gruppe und der Anzahl toter Bakterienzellen für die erste Gruppe entspricht.

6. Verfahren nach Anspruch 5 oder 6, **dadurch gekennzeichnet, dass** vor jeder Messung ferner ein drittes Fluorochrom, das nur dann ein Fluoreszenzsignal emittiert, wenn es mit einer lebenden Zelle reagiert, zu der gegebenenfalls verdünnten Probe hinzugefügt wird, dass die Probe so angeregt wird, um auch die Fluoreszenzemission des dritten Fluorochroms zu erhalten, und dass für die Punkte des zweiten und/oder dritten Fensters ein biparametrisches Histogramm erstellt wird, das für jeden Punkt die Intensität der Fluoreszenz aufgrund des Fluorochroms, das sich nur an die DNA von Zellen mit durchlässiger Wand bindet, und die Intensität der Fluoreszenz aufgrund des dritten Fluorochroms angibt, dann für jedes Fenster drei Untergruppen von Punkten bestimmt werden, wobei eine erste Untergruppe von Punkten einer Fluoreszenzintensität aufgrund des dritten Fluorochroms entspricht, die höher ist als die der anderen Untergruppen, wobei diese erste Untergruppe von Punkten die Zellen von *Brettanomyces* spp./lebenden und aktiven bakteriellen Zellen darstellen, wobei eine zweite Untergruppe von Punkten einer Fluoreszenzintensität aufgrund des dritten Fluorochroms entspricht, die niedriger ist als die der ersten Untergruppe und mit einer Fluoreszenzintensität aufgrund des ersten/zweiten Fluorochroms, die niedriger ist als die der dritten Untergruppe, gekoppelt ist, wobei die Punkte dieser zweiten Untergruppe den Zellen von *Brettanomyces* spp./Bakterienzellen im latenten Zustand entsprechen, und wobei eine dritte Untergruppe von Punkten einer Fluoreszenzintensität aufgrund des ersten/zweiten Fluorochroms entspricht, die höher ist als die der ersten und zweiten Untergruppe, wobei diese Punkte den Zellen von *Brettanomyces* spp./toten Bakterienzellen entsprechen.

7. Verfahren nach einem der vorhergehenden Ansprüche, **dadurch gekennzeichnet, dass** das erste und das zweite Fluorochrom unterschiedlich sind und aus Fluorochromen, die geeignet sind, sich an die DNA von Zellen zu binden, und eine maximale Fluoreszenzabsorptionswellenlänge von größer gleich 599 nm und von kleiner gleich 657 nm, eine maximale Fluoreszenzemissionswellenlänge von größer gleich 619 nm und von kleiner gleich 678 nm und eine Quantenausbeute von größer gleich 0,16 und von kleiner gleich 0,39 aufweisen, und deren Mischungen ausgewählt sind, insbesondere aus Fluorochromen, die geeignet sind, sich an die DNA von Zellen zu binden und eine maximale Fluoreszenzabsorptionswellenlänge von 652 nm, eine maximale Fluoreszenzemissionswellenlänge von 676 nm und eine Quantenausbeute der Fluoreszenz an DNA von 0,27 aufweisen, und Fluorochromen, die geeignet sind, sich an DNA zu binden, und eine maximale Fluoreszenzabsorptionswellenlänge von 657 nm, eine maximale Fluoreszenzemissionswellenlänge von 673 nm und eine Quantenausbeute der Fluoreszenz an DNA von 0,17 aufweisen, Fluorochromen, die geeignet sind, sich nur an die DNA von Zellen mit durchlässiger Wand zu binden, und eine maximale Fluoreszenzabsorptionswellenlänge von 547 nm, eine maximale Fluoreszenzemissionswellenlänge von 570 nm und eine Quantenausbeute der Fluoreszenz an der DNA von 0,9 aufweisen, und deren Mischungen, und dass, wenn das erste Fluorochrom aus den Fluorochromen, die geeignet sind, sich an die DNA zu binden, und eine maximale Fluoreszenzabsorptionswellenlänge von 657 nm aufweisen, eine maximale Fluoreszenzemissionswellenlänge von 673 nm und eine Quantenausbeute der Fluoreszenz an DNA von 0,17 aufweisen, und Fluorophoren ausgewählt ist, die geeignet sind, sich an die DNA von Zellen zu binden, und eine maximale Fluoreszenzabsorptionswellenlänge von 652 nm, eine maximale Fluoreszenzemissionswellenlänge von 676 nm und eine Quantenausbeute der Fluoreszenz an DNA von 0,27 aufweisen, das zweite Fluorochrom aus Fluorochromen ausgewählt ist, die geeignet sind, sich nur an die DNA von Zellen mit durchlässiger Wand zu binden, und eine maximale Fluoreszenzabsorptionswellenlänge von 547 nm und eine maximale Fluoreszenzemissionswellenlänge von 570 nm und eine Quantenausbeute der Fluoreszent an DNA von 0,9 aufweisen, und dass das dritte Fluorochrom unter 5-Carboxyfluoresceindiacetat, 6-Carboxyfluoresceindiacetat ausgewählt ist, Gemische aus 5-Carboxyfluoresceindiacetat und 6-Carboxyfluoresceindiacetat und 5,6-Carboxylatfluorescenindiacetat-succinimidylester der folgenden allgemeinen Formel (1) ausgewählt ist:

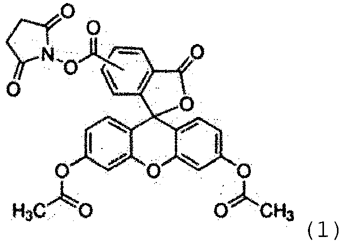

8. Verfahren nach einem der vorhergehenden Ansprüche, **dadurch gekennzeichnet, dass** das Substrat aus Schaumwein oder stillem Wein, Rotwein, Weißwein, Roséwein, Apfelwein, Bier, Sake, Fruchtsäften, insbesondere aus Trauben- oder Apfelsaft, Wasserkefir, Fruchtsaftkefir, Milchkefir, Milch, Tequila, Whisky, Wodka, Most, insbesondere Traubenmost, Weinen der ersten oder zweiten Gärung, fertigen Schaumoder stillen Weinen und Essigen ausgewählt ist.

9. Verfahren nach einem der vorhergehenden Ansprüche, **dadurch gekennzeichnet, dass** es das Nachweisen, Quantifizieren und Unterscheiden mindestens einer Hefeart von *Brettanomyces spp.* ermöglicht, die aus den folgenden Arten *B. anomalus, B. bruxellensis, B. custersianus, B. nanus, B. dekkera bruxellensis und B. naardenensis* von mindestens einer anderen Hefeart und insbesondere von mindestens einer Art von *Saccharomyces spp.* ausgewählt ist, ausgewählt aus den folgenden Arten: *Saccharomyces bailii Linder, Saccharomyces carlsbergensis, Saccharomyces cerevisiae, Saccharomyces delbrueckii, Saccharomyces exiguus, Saccharomyces fermentati, Saccharomyces florentinus, Saccharomyces fragilis, Saccharomyces fructuum, Saccharomyces heterogenicus, Saccharomyces oleaginosus, Saccharomyces rosei, Saccharomyces steineri, Saccharomyces boulardii, Saccharomyces kefir, Saccharomyces kluyveri* und insbesondere *Saccharomyces cerevisiae.*

10. Verfahren nach einem der vorhergehenden Ansprüche, **dadurch gekennzeichnet, dass** die Probe bei einer Wellenlänge von größer gleich 620 nm und von kleiner gleich 750 nm einschließlich und insbesondere von gleich 637 nm angeregt wird.

## Claims

1. A method for detecting, quantifying, and discriminating cells of *Brettanomyces spp* yeasts contained in an organic liquid substrate which contains fermentable sugars, wherein a sample of said substrate is taken, possibly diluted, and at least one first fluorochrome capable of binding to DNA of both dead and/or living cells is added to the possibly diluted substrate, said sample is then irradiated so as to obtain fluorescence emission from the first fluorochrome, and said sample is also irradiated to obtain fluorescence emission from the sample at 670 nm, a dual histogram is established, giving for each point the fluorescence intensity due to the first fluorochrome and the fluorescence intensity emitted at 670 nm, at least one first point cloud corresponding to a fluorescence intensity emitted and detected at 670 nm that is higher than that detected for the other points, it is deduced that the points in said first cloud correspond to the cells of *Brettanomyces* spp, and the number of *Brettanomyces spp* cells is optionally counted by counting the points in said first cloud.

2. The method according to claim 1, **characterised in that** said substrate contains mostly *Brettanomyces spp* yeasts and Sacharomyces spp yeasts, **in that** two point clouds are obtained on said dual histogram, a first cloud comprising the points corresponding to a fluorescence intensity emitted at 670nm greater than that of the points of the second point cloud, **in that** it is deduced therefrom that the points of said first cloud correspond to the *Brettanomyces spp* cells and that the points of said second cloud correspond to the *Saccharomyces spp* cells and the number of *Brettanomyces spp* and/or Saccharomyces spp cells is optionally counted by counting the points of each of said clouds.

3. The method according to claim 1 or 2, **characterised in that** before measuring fluorescence, a first discrimination is performed between the particles and the cells present in the sample by measuring intensity of the reflected and refracted light as well as intensity of the diffracted light, a dual histogram is then established, giving the values of said intensities for each point corresponding to a detected particle or cell, based on the values of said intensities, a first window that contains points attributable to yeast cells, and optionally a second window corresponding to points attributable to bacterial cells are thus determined, said dual histogram giving fluorescence intensity due to the first fluorochrome and fluorescence intensity emitted at 670 nm for each point located in said first window is established.

4. The method according to any one of the preceding claims, **characterised in that** the first fluorochrome is capable of binding to DNA of live cells as well as to DNA of cells whose wall is permeable, **in that** prior to any measurement, a second fluorochrome capable of binding only to DNA of cells whose wall is permeable, is additionally added to the possibly diluted sample, **in that** a third window is further determined that surrounds the points of said first cloud, **in that** the sample is excited to cause fluorescence emission from the first and second fluorochromes, and for points located in said third window, a dual histogram is also generated, giving for each point the fluorescence intensity of the first and second fluorochromes, or the fluorescence intensity per unit area of one of the two fluorochromes and that due to the other fluorochrome, and **in that** two groups of points are further determined, a first group for which fluorescence due to the fluorochrome that binds solely to DNA of cells whose wall is permeable is greater than that of the second group, and the number of points in each group is counted, which corresponds to the number of live *Brettanomyces spp* cells for the second group and the number of dead *Brettanomyces spp* cells for the first group.

5. The method according to claims 2 and 3, **characterised in that** said second window is determined and that said sample is also excited so as to cause fluorescence emission from both the first and second fluorochromes, and for points located in said second window, a dual histogram is established, giving fluorescence intensity for each point of both the first and second fluorochromes, or the fluorescence intensity per unit area of one of both fluorochromes and that due to the other fluorochrome, and **in that** two groups of points are identified, a first group for which fluorescence due to the fluorochrome that binds only to DNA of cells whose wall is permeable is greater than that of the second group, and the number of points in each group is then counted, which corresponds to the number of live bacterial cells for the second group and the number of dead bacterial cells for the first group.

6. The method according to claims 5 or 6, **characterised in that**, prior to any measurement, a third fluorochrome which emits a fluorescence signal only when it reacts with a living cell is added to the possibly diluted sample, the sample is then excited so as to obtain fluorescence emission of said third fluorochrome, and for points of the second and/or third window, a dual histogram giving, for each point, fluorescence intensity due to the fluorochrome that binds only to DNA of cells whose wall is permeable, as well as fluorescence intensity due to the third fluorochrome is established, for each window, three subgroups of points are then determined, a first subgroup of points corresponds to a fluorescence intensity due to the third fluorochrome greater than that of the other subgroups, this first subgroup of points representing *Brettanomyces spp* cells/live and active bacterial cells, a second subgroup corresponding to fluorescence intensity due to the third fluorochrome lower than that of the first subgroup and coupled with a fluorescence intensity due to the first/second fluorochrome lower than that of the third subgroup, the points of this second subgroup corresponding to *Brettanomyces spp* cells/bacterial cells in a latent state, and a third subgroup of points corresponding to fluorescence intensity due to the first/second fluorochrome that is greater than that of the first and second subgroups, these points corresponding to dead *Brettanomyces spp* cells/bacterial cells.

7. The method according to any one of the preceding claims, **characterised in that** said first and said second fluorochromes are different and selected from fluorochromes capable of binding to DNA of cells and having a maximum fluorescence absorption wavelength of 599 nm or greater and 657 nm or less, a maximum fluorescence emission wavelength of 619 nm or greater and 678 nm or less, and a quantum yield of 0.16 or greater and 0.39 or less, and mixtures thereof, in particular, the fluorochromes capable of binding to DNA of cells and having a maximum fluorescence absorption wavelength of 652 nm, a maximum fluorescence emission wavelength of 676 nm, and a fluorescence quantum yield on DNA of 0.27, and fluorochromes capable of binding to DNA and having a maximum fluorescence absorption wavelength of 657 nm, a maximum fluorescence emission wavelength of 673 nm, and a fluorescence quantum yield on DNA of 0.17, the fluorochromes capable of binding only to DNA of cells whose wall is permeable and which have a maximum fluorescence absorption wavelength of 547 nm, a maximum fluorescence emission wavelength of 570 nm, and a quantum fluorescence yield on DNA of 0.9, and mixtures thereof, and **in that** when the first fluorochrome is selected from fluorophores capable of binding to DNA with a maximum fluorescence absorption wavelength of 657 nm, a maximum fluorescence emission wavelength of 673 nm, and a fluorescence quantum yield on DNA of 0.17, and fluorophores capable of binding to DNA of the cells and having a maximum fluorescence absorption wavelength of 652 nm, a maximum fluorescence emission wavelength of 676 nm, and a fluorescence quantum yield on DNA of 0.27, the second fluorochrome is selected from fluorophores capable of binding only to DNA of cells whose wall is permeable, and which a maximum fluorescence absorption wavelength of 547 nm and a maximum fluorescence emission wavelength of 570 nm, and a quantum fluorescence yield on DNA of 0.9, and **in that** said third fluorochrome is selected from 5-carboxyfluorescein diacetate, 6-carboxyfluorescein diacetate, mixtures of 5-carboxyfluorescein diacetate and 6-carboxyfluorescein diacetate, and succinimidyl 5,6 carboxylate fluorescein diacetate of the following general formula (1):

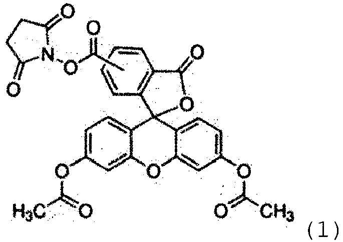

8. The method according to any one of the preceding claims, **characterised in that** said substrate is selected from sparkling or still wine, red wine, white wine, rosé wine, cider, beer, sake, fruit juices, particularly grape or apple juice, water kefir, fruit juice kefir, milk kefir, milk, tequila, whisky, vodka, musts, especially grape musts, wines undergoing primary or secondary fermentation, finished wines, whether sparkling or still, and vinegars.

9. The method according to any one of the preceding claims, **characterised in that** it enables quantification and discrimination of at least one *Brettanomyces spp* yeast species selected from the following species: *B. anomalus, B. bruxellensis, B. custersianus, B. nanus, B. dekkera bruxellensis and B. naardenensis* from at least one other yeast species and especially at least one *Saccharomyces spp* species selected from the following species: *Saccharomyces bailii Linder, Saccharomyces carlsbergensis, Saccharomyces cerevisiae, Saccharomyces delbrueckii, Saccharomyces exiguus, Saccharomyces fermentati, Saccharomyces florentinus, Saccharomyces fragilis, Saccharomyces fructuum, Saccharomyces heterogenicus, Saccharomyces oleaginosus, Saccharomyces rosei, Saccharomyces steineri, Saccharomyces boulardii, Saccharomyces kefir, Saccharomyces kluyveri* and in particular *Saccharomyces cerevisiae.*

10. The method according to any one of the preceding claims, **characterised in that** said sample is excited at a wavelength of greater than or equal to 620 nm and less than or equal to 750 nm inclusive, and in particular equal to 637 nm.
